# EUROPEAN PATENT APPLICATION

(11) **EP 4 603 826 A1**
(43) Date of publication of application: **20.08.2025**
(21) Application number: 24158304.6
(22) Date of filing: 19.02.2024
(51) Int. Cl.: G01N 21/89, G01N 33/36

(54) **YARN QUALITY DETECTION DEVICE AND METHOD**

(71) Applicant: Gebrüder Loepfe AG, 8623 Wetzikon (CH)
(72) Inventor: Sergeyev, Anton, 8049 Zurich (CH); Frick, Manuel Bruno, 8610 Uster (CH)
(74) Representative: E. Blum & Co. AG

(57) **Abstract**

An optical sensor device for detecting defects in an elongate textile body. The optical sensor comprising a measurement chamber (10) for the elongate textile body, a support body (14) having opposing first and second sides (24a, 24c) as well as peripheral sides extending transversally to said first and second sides (24a, 24c), at least a first light source, and at least a first and a second light detector. The first light source and the second light detector are both arranged on a first side (24a) of said measurement chamber (10). The first light detector is arranged on a second side (24c) of said measurement chamber (10). Said second side (24c) of said measurement chamber (10) is opposite to said first side (24a) of said measurement chamber (10).

## Description

### Technical Field

The invention relates to an optical sensor device for detecting defects in an elongate textile body as well as to a use of such a device.

### Background Art

Optical sensor devices for detecting defects in elongate textile bodies are known, for example, for detecting changes in thickness of a yarn or yarn precursor or for detecting foreign fibers in such a body. Optically detecting defects in elongate textile bodies poses challenges, in particular for the accurate identification of defects. For instance, different defects may be difficult to distinguish.

US 5414520 and EP 1508797 describe optical sensor devices for measuring foreign material. They propose using two light sources and three light detectors arranged around the textile body in order to detect foreign material therein. At least one of the detectors is used for measuring transmitted light in order to estimate the thickness of the yarn while at least one other detector is used to detect light reflected from the yarn.

EP3748343A1 describes an optical sensor device for detecting foreign material in yarn. This type of sensor is, e.g., used in quality control and monitoring for detecting contaminants in the elongate body, such as foreign fibers or vegetable matter.

Since the reflected light depends on both the thickness of the yarn as well as the presence of contaminations, a combined measurement of reflection and transmission improves the measurement reliability.

### Disclosure of the Invention

It is an objective of the present invention to provide an optical sensor device for detecting defects in an elongate textile body with improved reliability.

The objective is achieved by the subject-matter of the independent claims. Other advantageous embodiments are listed in the dependent claims as well as in the description below.

An aspect of the invention relates to an optical sensor device for detecting defects in an elongate textile body. The device comprises:
- A support body: The support body may consist of a single body. Alternatively, the support body may comprise several sub-bodies, in particular several interconnected sub-bodies. The support body may comprise or consist of a polymer. The support body may have the shape of a U or a V.
- A measurement chamber for receiving the elongate textile body running along a direction X: The measurement chamber is, along the direction X, open on opposite entry and exit sides. In addition, the measurement chamber is, along a direction Y transversal to the direction X, confined by the support body on a first and second side.
- At least one measurement window arranged on the first side, wherein, along the direction X, the support body comprises frame elements adjacent to the measurement window.
- An optical transmission sensor comprising a transmission light source arranged to send light through the measurement chamber: The optical transmission sensor further comprises a transmission light detector arranged to receive light from the transmission light source after traversing the measurement chamber and the window. The transmission light may first traverse the measurement chamber and then the window, or the transmission light may first traverse the window and then the measurement chamber. The measurement chamber is arranged between the transmission light source and the transmission light detector.
- An optical reflection sensor comprising a reflection light source arranged to send light into the measurement chamber: The optical reflection sensor further comprises a reflection light detector arranged to receive light from the reflection light source after being reflected in the measurement chamber. The reflection light source and the reflection light detector are both arranged at the second side. The reflection light source is positioned to illuminate at least an illuminated section of the frame elements. The reflection light detector is positioned to receive light from the illuminated section.

The support body allows to improve the stability of the optical sensor.

The direction X, the direction Y and the direction Z may be transversal to each other, in particular they may be perpendicular to each other. Two directions are transversal if they are non-parallel, in particular perpendicular, to each other.

The measurement chamber may have a cuboid shape.

The distance in direction Y between the first and the second side may be between 0.1 cm and 2 cm, in particular between 0.2 cm and 1 cm. This allows the elongate textile body to move along the direction X through the measurement chamber, in particular to move along the direction X without rubbing on the first and the second side while keeping the measurement volume low enough for accurate measurements.

The transmission light source and the reflection light source may be the same light source or different light sources. In general, the transmission sensor and the reflection sensor may share one or more light sources and/or light detectors.

In one embodiment, at least 50% of the light emitted from the transmission light source and entering the measurement window reaches the transmission light detector.

The measurement window allows to spatially restrict (or select) which light emitted by the transmission light source reaches the transmission light detector. This allows to define a transmission measurement region in the measurement chamber where the transmission light reaching the transmission light detector passes, thereby potentially improving the signal to noise ratio by reducing the amount of light that did not interact with the elongate textile body. Also, the transmission window may be positioned to only transmit light from a region of the measurement chamber that is illuminated homogeneously by the transmission light source. In this context, a homogeneous illumination is understood to be an illumination where the illumination intensity varies by less than 10%, in particular by less than 5%.

Transmission measurements allow to accurately measure thickness defects in elongate textile bodies. In a transmission measurement, the light emitted by the transmission light source and reaching the transmission light detector is measured, wherein the actual signal is the light "missing" due to the presence of the elongate textile body in a path of the transmission light.

The reflection light source may be arranged to send light into the measurement chamber to a region at least partially overlapping with the transmission measurement region.

Advantageously, the transmission light source and the reflection light source are arranged to both send light onto the measurement window, in particular onto the elongate textile body in front of the measurement window. (In this context, "in front of the measurement window" is to be understood as designating a location on the chamber-side of the measurement window.) This allows to perform reflection measurements and transmission measurements on the same location/spot on the elongate textile body.

The elongate textile body may be a yarn.

The frame elements adj acent to the measurement window may, in the direction X, extend from the measurement window to an edge of the support body.

The frame elements may comprise or consist of a different material than the material of the remainder of the support body.

The transmission light source and/or the reflection light source may be a semiconductor device that emits light, in particular a light-emitting diode.

In one embodiment, the transmission light source and/or the reflection light source has a low spectral coherence compared to a diode laser. In particular the transmission light source and/or the reflection light source is not spectrally coherent. This allows to reduce interference effects, which in particular improves measurement quality.

Advantageously, the optical reflection sensor comprises at least two reflection light detectors. The at least two reflection light detectors of the optical reflection sensor may be arranged on the second side. The at least two reflection light detectors may be arranged to view the measurement window under different angles. This allows for a more uniform observation and, in particular, therefore improves the reflection measurements.

Advantageously, the frame elements have a reflectance of at most 80 %, in particular at most 50 %, more particularly at most 20 %, over an emission spectrum of the reflection light source. If there are several reflection light sources, this condition is advantageously met for every one of them.

Measuring the reflection from the elongate textile body may allow to gain a correlated color and thickness information about the elongate textile body. Measuring the transmission of the elongate textile body may allow to gain thickness information (uncorrelated) about the elongate textile body. Combining the measured reflection and transmission of the elongate textile body may allow to gain information on the color that is less dependent on the thickness than the reflection alone. To do so, it is advantageous to measure both the reflection and transmission over the same region/area of the elongate textile body.

If the reflection light source illuminates a peripheral area outside the measurement window, in particular the frame elements, the reflection measurement is influenced by the reflection from said peripheral area. In particular, the measured reflection is the sum of a core component reflected from an area that is also monitored by the transmission sensor and of a peripheral component from the peripheral area. The peripheral component may depend on thickness variations of the textile body in the background area, which makes it harder to compensate the measured reflection using the transmission measurement.

This is particularly apparent for dark textile bodies with low reflectivity in combination with conventional sensor devices having a support body with high reflectance. In this case, an increase of the thickness of the body in the peripheral area will reduce the measured reflection but not affect the measured transmission, which makes the system incorrectly assume that the textile body has a "darker" area.

Therefore, using frame elements with a reflection of at most 80 %, in particular at most 50 %, more particularly at most 20 %, over the emission spectrum of the reflection light source allows to improve the measurement quality and/or reliability.

The reflectance of the frame elements may be reduced by using dark-colored frame elements. The frame elements may be black.

Advantageously, an average reflectance of the frame elements over a spectral range ranging from 400 nm to 780 nm is at most 80 %, in particular at most 50 %, more particularly at most 20 %.

In this context an average reflectance over a spectral range ranging from 400 nm to 780 nm is to be understood as the average of the reflectance for each individual wavelength over the full range from 400 nm to 780 nm.

This allows to reduce the visible light (i.e., 400 nm to 780 nm) reflected on the frame elements, which reduces variations in the reflection signal due to thickness variations of the elongate textile body outside the measurement window but still illuminated by the reflection light source. This allows to improve the assessment of the visible quality of the textile body, in particular to improve the determination of the color of the elongate textile body.

This is based on the understanding that it is sufficient to have low reflectance over the spectrum of the reflection light source. If there are several reflection light sources having different emission spectra, the average reflectance is advantageously calculated over the mission spectrum of each of them.

In this context, the average reflectance over the emission spectrum of the light source is the reflectance calculated as the average over said spectrum weighted with the relative intensity distribution of the light in the emission spectrum.

Advantageously, the frame elements have a bulk material and a surface layer. The surface layer may have an average reflectance of at most 80 %, in particular at most 50 %, more particularly at most 20 %, over the emission spectrum of the reflection light source. In particular, the frame element may have an average reflectance over said emission spectrum that is lower than the one of the bulk material.

Advantageously, the bulk material of the frame element is a material having an average reflection over the emission spectrum of the reflection light source of at least 80%, in particular of a least 90%, which reduces light losses as the light traverses openings in the frame element.

The surface layer may have a thickness of less than a millimeter. The surface layer may be a layer of paint, in particular of black paint. In another embodiment, the surface layer may be formed by a surface treatment that changes the optical properties of the bulk material. For example, laser irradiation can, e.g., be used to oxidize the bulk material and generate a surface layer having less reflection.

Advantageously, the transmission light sensor is arranged on the first side.

This allows for a compact design. In particular, having the reflection light source and the reflection light detector both arranged at the second side and the transmission light sensor arranged at the first side allows to use the reflection light source for both reflection measurements and transmission measurements, i.e., the reflection light source and the transmission light source are formed by the same light source.

Advantageously, the frame elements have a width, in the direction X, of at most 1.5 mm, in particular of at most 0.5 mm. In this context, the width of the frame elements is measured in the middle of the measurement field (i.e., where the elongate textile body is typically most likely to be).

The frame elements having a width, in the direction X, of at most 1.5 mm or even smaller allows to reduce the surface onto which the light emitted by the reflection light source may be reflected, therefore reducing the peripheral component of the reflection.

Advantageously, at least one of the frame elements has an inclined surface that faces the measurement chamber and that is positioned to be illuminated by the reflection light source, in particular by at least one of the light sources. This inclined surface is non perpendicular to the direction Y. In particular, said inclined surface forms an angle between 2 to 88 degrees to the direction Y, more particularly forms an angle between 10 to 70 degrees to the direction Y.

The inclined surface is facing the measurement chamber (i.e., it is not on the lateral surfaces of the frame elements or on the surface of the frame elements facing away from the measurement chamber).

Such an inclined surface allows to reduce the amount of reflected light from the reflection light source that reaches the reflection light detector. Therefore, this allows to improve measurement quality.

Advantageously, the inclined surface is such that at least 50% of the light from the reflection light source is specularly reflected therefrom. The inclined surface, may, for example be smooth and have an absorption of at most 30% in the range from 400 nm to 780 nm.

The inclined surface may be flat or curved. Advantageously, in the case of a curved inclined surface, at least part of the curved surface forms said angle with the direction Y.

Advantageously, the inclined surface forms at least 20%, in particular at least 50%, of the total surface of the frame elements that is positioned to be illuminated by the reflection light source.

In one embodiment, the optical reflection sensor comprises at least two, in particular at least three, reflection light sources having different emission spectra, in particular having non overlapping emission spectra.

The at least three reflection light sources may comprise or consist of a blue reflection light source, a red reflection light source and a green reflection light source. The blue reflection light source may have a full-width-half maximum (FWHM) emission wavelength range between 440 nm and 500 nm. The green reflection light source may have a FWHM emission wavelength range between 510 nm and 570 nm. The red reflection light source may have a FWHM emission wavelength range between 600 nm and 750 nm.

In another embodiment, the optical reflection sensor comprises at least one broad-band reflection light source having an FWHM emission spectrum extending over at least 200 nm.

Advantageously, the support body forms walls of channels extending between the chamber and at least some of the light sources and light detectors. The average reflectance of said walls over the emission spectrum of the reflection light source is higher than the average reflectance of said frame elements over the emission spectrum of the reflection light source.

Such comparatively reflective walls allow the light detector to receive more light.

Advantageously, the support body forms walls of channels extending between the measurement window of the first side and a light detector on the first side, in particular the transmission light detector on the first side.

Advantageously, the walls of channels are white. The walls of channels may be flat and smooth.

Advantageously, the reflectance of these walls is at least twice as large as the reflectance of the frame elements.

The reflectance of the walls may be at least 60%, in particular at least 75%, more particularly at least 90 %.

Advantageously, said "reflectance" of the previous two paragraphs is the average reflectance over the spectral range of at least one of the light sources, in particular of at least all of the light sources.

This allows to minimize losses of light (light intensity) before reaching the light detectors (reflection or transmission light detector) and therefore to improve the measurement quality.

The invention also relates to the use of the optical sensor device as described above to perform measurements on an elongated textile body having an average reflectance of at most 80% over the emission spectrum of the reflection light source, in particular an average reflectance of at most of at most 50 %, more particularly at most 20 %.

The elongate textile body may have a black color.

Advantageously, the elongate textile body has an average reflectance of at most 80% over the emission spectrum of any one of the reflection light sources of the optical reflection sensor, in particular an average reflectance of at most of at most 50 %, more particularly at most 20 %.

### Brief Description of the Drawings

The invention will be better understood and objects other than those set forth above will become apparent from the following detailed description thereof. Such description makes reference to the annexed figures. These figures show:
Fig. 1 shows a representation of an optical sensor device cut along its middle,
Fig. 2 shows a sectional view along line II-II of Fig. 1,
Fig. 3 Shows another example of an optical sensor device cut along its middle,
Fig. 4 shows yet another example of a sensor device,
Fig. 5 is a sectional view along line IV-IV of Fig. 4,
Fig. 6 shows a schematic representation of a defect of the elongate body in front of the measurement window,
Fig. 7 shows a schematic representation of a defect of the elongate body in front of the frame elements,
Fig. 8 shows an embodiment with collimation optics,
Fig. 9 shows an embodiment with a reflector,
Fig. 10 shows an embodiment where the window element covers the frame elements,
Fig. 11 shows an embodiment with multi-section frame elements.

### Modes for Carrying Out the Invention

### Embodiment 1

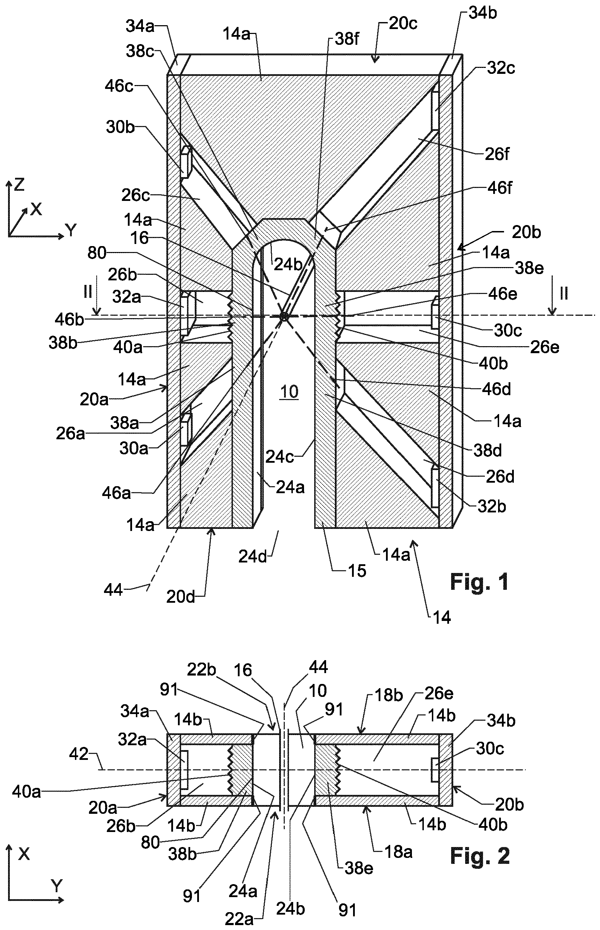

Figs. 1 and 2 show a schematic representation of an optical sensor device for detecting defects in an elongate textile body cut along its middle.

The device comprises a slit-shaped measurement chamber 10 formed by a slit in a support body 14. Measurement chamber 10 is designed to receive the elongate textile body 16 running, during the measurement, along a direction X.

Support body 14 has a first exterior side 18a, a second exterior side 18b, which are advantageously parallel to each other (see Fig. 2) and transversal to direction X. It further has peripheral sides 20a, 20b, 20c, and 20d (see Figs. 1 and 2) extending transversally, in particular perpendicularly, to first and second exterior side 18a, 18b.

Measurement chamber 10 is open, along direction X, at first exterior side 18a and second exterior side 18b, where it forms an entry side 22a, and an exit side 22b, through which elongate textile body 16 can enter and exit during the measurement.

At its sides perpendicular to the first and second exterior side 18a, 18b, measurement chamber 10 is closed on a first side 24a, a second side 24c, and a third side 24b by support body 14 while it is open at a fourth side 24d for inserting elongate textile body 16 before operation.

In other words, along a direction Y transversal, in particular perpendicular, to the direction X, measurement chamber 10 is confined by the support body on the first and second sides 24a, 24c. Further, along a direction Z perpendicular to the directions X and Y, measurement chamber 10 is closed at third side 24b while being open at fourth side 24d.

Radial light channels 26a - 26f extend through support body 14 between measurement chamber 10 and a first peripheral side 20a and a second peripheral side 20b.

Advantageously, first peripheral side 20a and second peripheral side 20b are located opposite to each other, and in particular they are parallel to each other.

The sensor device further comprises several light sources 30a, 30b, 30c and several light detectors 32a, 32b, 32c. In the present embodiment, there are three light sources as well as three light detectors. As mentioned above, that number may be larger.

The light sources 30a, 30b, 30c and the light detectors 32a, 32b, 32c are alternatingly arranged at the light channels 26a - 26f, namely at the first and second peripheral sides 20a, 20b of support body 14.

The light sources 30a, 30b, 30c and the light detectors 32a, 32b, 32c are mounted on first and second carrier plates 34a, 34b.

First carrier plate 34a is mounted to first peripheral side 20a of support body 14. It carries the light sources 30a, 30b as well as the light detector 32a arranged on that side. Second carrier plate 34b is mounted to second peripheral side 20b of support body 14. It carries the light source 30c as well as the light detectors 32b and 32c arranged on that side.

The carrier plates 34a, 34b are advantageously printed circuit boards and they are arranged parallel to each other. They may be mechanically connected to support body 14 in defined spatial relation such that the light sources and light detectors are located at the desired positions in the light channels 26a - 26f.

As can be seen, there is a first light source 30a and a second light source 30b as well as a first light detector 32a arranged on the first side 20a, and there is a third light source 30c as well as a second light detector 32b and a third light detector 32c arranged on the second side.

The first light source 30a is opposite the third light detector 32c, the second light source 30b is opposite the second light detector 32b, and the third light source 30c is opposite the first light detector 32a. In this context, "opposite" means on opposing sides of the nominal position of elongate body 16 in measurement chamber 10 (i.e. the center of measurement chamber 10).

In more general terms, each light detector may be arranged opposite from one of the light sources and vice versa.

As mentioned above, at least one light detector 32a is arranged between at least two light sources 30a, 30b on a first side of measurement chamber 10, and at least one light source 30c is arranged between at least two light detectors 32b, 32c on a second side of measurement chamber 10.

For a reflection measurement, the third light source 30c may be used as reflection light source and the second and/or third light detectors 32b, 32c may be used as reflection light detectors, i.e., the sensor device has an optical reflection sensor comprising third light source 30c as well as second and/or third light detector(s) 32b, 32c. Alternatively or in addition, the first and second light sources 30a, 30b may be used as reflection light sources and the first light detector 32a may be used as reflection light detector, i.e., the sensor device has an optical reflection sensor comprising the first and second light sources 30a, 30b as well as first light detector 32a.

For a transmission measurement, the third light source 30c may be used as transmission light source and the first light detector 32a may be used as transmission light detector. In other words, the sensor device has an optical transmission sensor comprising third light source 30c and first light detector 32a.

Typically, the light detectors 32a - 32c have a larger active (i.e. light-sensitive) area than the active (i.e. light-emitting) area of the light sources 30a - 30c. It is advantageous that the light sources and light detectors have equal active area. To compensate for this area inequality at least in part, support body 14 is designed such that first peripheral side 20a (and the light sources and light detectors arranged there) is closer to measurement chamber 10 than second peripheral side 20b (and the light sources and light detectors arranged there).

The shown embodiment has a first light channel 26a extending between first light source 30a and measurement chamber 10, a second light channel 26b extending between first light detector 32a and measurement chamber 10, a third light channel 26c extending between second light source 30b and measurement chamber 10, a fourth light channel 26d extending between second light detector 32b and measurement chamber 10, a fifth light channel 26e extending between third light source 30c and measurement chamber 10, and a sixth light channel 26f extending between third light detector 32c and measurement chamber 10.

Hence, the first, second, and third light channel 26a - 26c extend through support body 14 between measurement chamber 10 and first peripheral side 20a. The fourth, fifth, and sixth light channel 26d - 26f extend through support body 14 between measurement chamber 10 and second peripheral side 20b.

The second light channel 26b is arranged between the first light channel 26a and the third light channel 26c. Similarly, the fourth light channel 26e is arranged between the fourth light channel 26d and the sixth light channel 26f.

The interface of the second light channel 26b with the measurement chamber, on the first side 24a, forms a measurement window 80.

The second light channel 26b and the fifth light channel 26e are coaxial, such that light source 30c is arranged exactly vis-à-vis light detector 32a in order to provide an accurate transmission measurement of measurement chamber 10. This allows to generate a signal dependent on the thickness of elongate object 16.

Support body 14 comprises first sub-bodies 14a arranged, along direction X, between two second sub-bodies 14b. The sub-bodies 14a, 14b form the light channels 26a - 26f between them. The second sub-bodies 14b form the exterior sides 18a, 18b.

The first body sub-bodies 14a are advantageously of a non-transparent material (i.e., a material not transparent in the spectral region of overlap between the emission of the light sources and the sensitivity of the light detectors), and they separate the light channels from each other in order to suppress optical crosstalk between them.

In more general terms, the light channels 26a - 26f (optically) advantageously communicate through the measurement chamber, i.e., a significant part of the light (i.e., at least 10%, in particular at least 25%) passing from one to the other has passed through measurement chamber 10.

Advantageously, and as mentioned above, the sub-bodies 14a, 14b are of a material that reflects the light from the light sources strongly.

The device further comprises a window element 15 of a transparent material (i.e., a material transparent in the spectral region overlap between the emission of the light sources and the sensitivity of the light detectors, in particular in the range from 400 nm to 780 nm). It forms transparent windows 38a - 38f between the light channels 26a - 26f and measurement chamber 10. These windows prevent dirt from entering the light channels 26a - 26f. Advantageously, the windows 38a - 38f are transparent in the sense that they let at least 50% of the light in the spectrum of overlap between the light sources and the light detectors pass.

The windows 38b and 38e form a first diffusor 40a and a second diffusor 40b arranged at the interface between second light channel 26b and measurement chamber 10 as well as at the interface between fourth light channel 26e and measurement chamber 10. They increase the precision of the measurement.

Diffusors 40a, 40b may e.g. be formed by small structures (having sizes between 10 and 500 µm) arranged on a surface of the windows 38b, 38e, in particular on a side of these windows that faces away from measurement chamber 10.

Advantageously, the sub-bodies 14a, 14b and window element 15 are manufactured by two-component casting or two-component molding. They may, e.g., also be formed by separate elements glued to each other.

The light channels 26a - 26f are advantageously hollow, i.e., filled with a gas, in particular air. In order to increase their light ducting properties, their inner walls (i.e., inner surfaces) are advantageously smooth and reflective.

Alternatively, the light channels 26a - 26f may be filled with a transparent solid.

As can be seen from Fig. 2, the light sources 30a - 30c, the light detectors 32a - 32c, and the measurement chamber 10 are arranged in a common plane 42 (i.e. they are intersected by said common plane). Advantageously, this common plane 42 extends parallel to first exterior side 18a and second exterior side 18b of support body 14 and/or perpendicular to direction X, i.e. to elongate direction 44 of elongate textile body 16.

In said common plane 42, the light sources 30a - 30c and the light detectors 32a - 32c are arranged at different angular positions. These angular positions are defined by the connecting lines between the nominal position of the elongate textile body 16 (i.e. the center of measurement chamber 10) and the respective light source or light detector.

Further, the windows 38a - 38f are arranged at different angular positions 46a - 46f around the center of measurement chamber 10.

### Embodiment 2

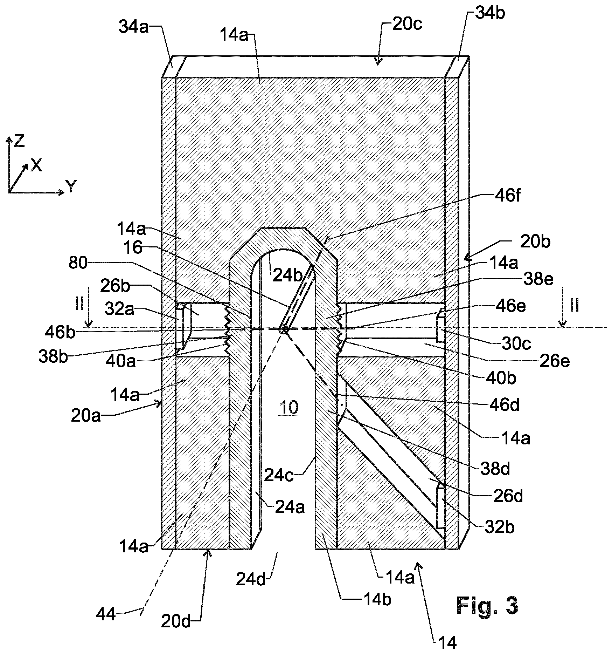

Figs. 3 shows a schematic representation of an optical sensor device cut along its middle.

The device is similar to the one shown in Fig. 1 and Fig. 2, however in this embodiment it has one light source 30c and two light detectors 32a, 32b. Therefore, the optical sensor device only has three radial light channels 26b, 26d, 26e.

The light source 30c is used as reflection light source and transmission light source. The light detector 32b is the reflection light detector 32b and the light detector 32a is the transmission light detector 32a.

Hence, in this embodiment, the transmission sensor comprises light source 30c as transmission light source and light detector 32a as transmission light detector, and the reflection sensor comprises light source 30c as reflection light source and light detector 32b as reflection light detector.

### Embodiment 3

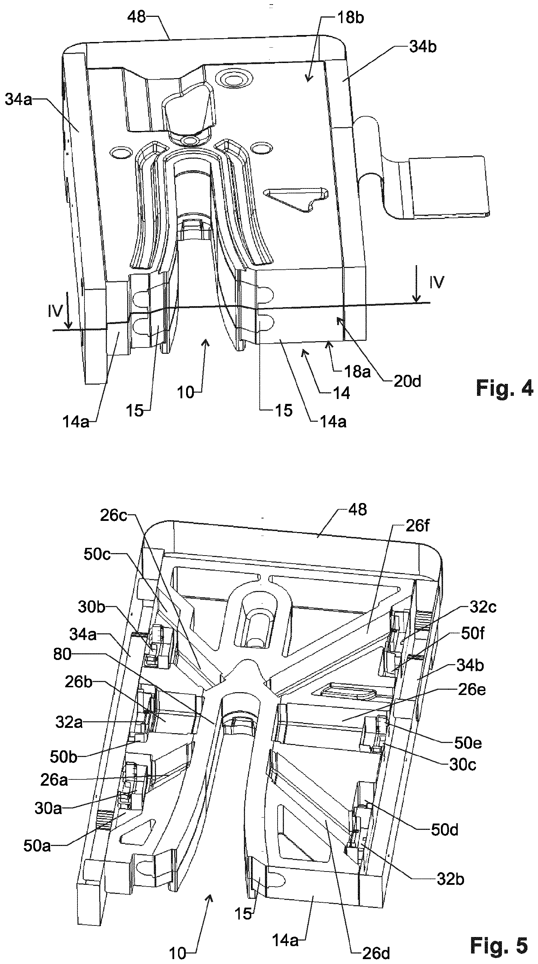

Figs. 4 and 5 show a specific embodiment of the sensor device.

As can be seen, measurement chamber 10 is formed by a slit of support body 14 that widens towards fourth peripheral side 20d.

Window element 15 forms part of the inner wall of measurement chamber 10. The non-transparent, second sub-bodies 14b form the first exterior side 18a and the second exterior side 18b of support body 14. They close the light channels 26a - 26f at these first and second exterior sides 18a, 18b.

The two carrier plates 34a, 34b are mounted to opposite peripheral sides of support body 14 and may e.g. be electrically interconnected by means of a flexible printed circuit 48.

At least some of the light sources 30a - 30c and/or at least some of the light detectors 32a - 32b are located in recesses 50a - 50f of support body 14, with each recess 50a - 50f forming an end of one light channel 26a - 26f.

### Frame elements

As mentioned above, one aspect of the present sensor device deals with the influence of the optical reflection on the frame elements 90 adjacent to the measurement window 80. This is an aspect that applies to all embodiments shown herein as well as to most other optical sensor devices of this type. It is discussed in more detail in the following.

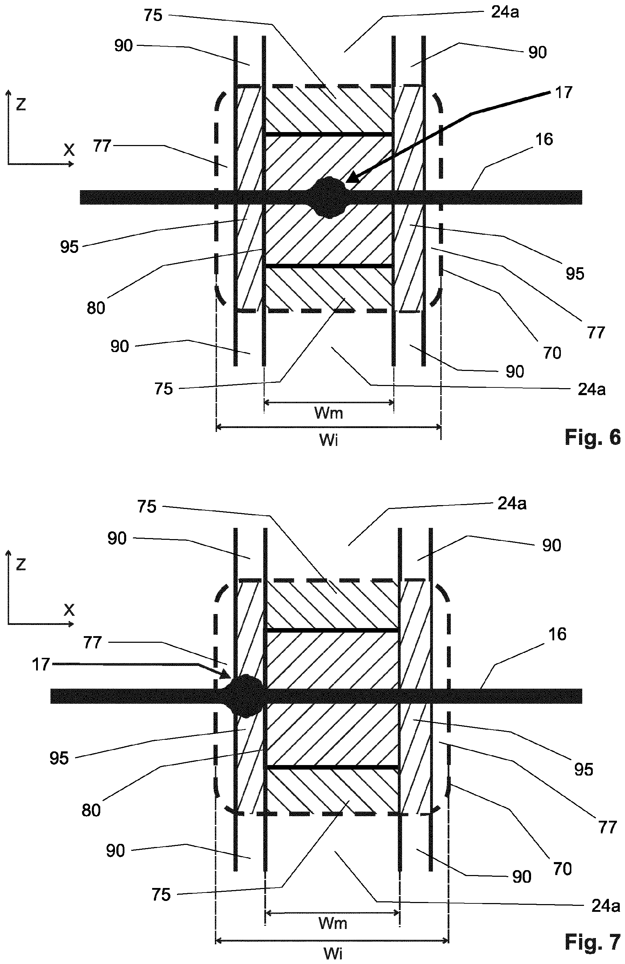

Fig. 6 shows a schematic representation of the region around measurement window 80 as seen when looking from the second side 24c onto the first side 24a of the measurement chamber 10.

Measurement window 80 is defined as the geometrical area at first side 24a that is traversed by the fraction of light from the transmission light source that will reach the transmission light detector.

The second sub-bodies 14b form frame-elements 90 that are, along direction X, laterally adj acent to measurement window 80.

In the above embodiments, the light from light source 30c, which is used as transmission light source, enters measurement chamber 10 through window element 15, traverses measurement chamber 10, leaves it through measurement window 80, traverses window element 15, and finally reaches light detector 32a, which acts as the transmission light detector.

The light detector 32a is located opposite the light source 30c as described for Fig. 1, Fig. 2 and Fig 3. The light source arrangement is as shown in Fig. 3 (embodiment 2). The light source 30c is the transmission light source and the reflection light source.

The light source 30c illuminates an illumination area 70 that has, along direction X, a width Wi larger than the width Wm of measurement window 80 along the same direction. The illumination area 70 covers the measurement window 80 but, beyond that, it also covers, or may cover, further regions at first side 24a:
- It illuminates a part 95 of the frame elements 90 adjacent, along direction X, to measurement window 80.
- It illuminates a part 75 of support body 14 adjacent, along direction Z, to measurement window 80.
- It may illuminate a part 77 of the measurement chamber outside support body 14.
- It illuminates part of the elongate textile body 16.

For reflection measurements, the reflection light detector, i.e., the second light detector 32b and/or the third light detector 32c, collects the light emitted by the light source onto the illumination area 70 and reflected onto the reflection light detector. The measured reflection R is the sum R = Rc + Rp of a core component Rc and of a peripheral component Rp.
- The core component Rc is generated by light reflected from the measurement window 80 including the part of the elongate textile body 16 in front of the measurement window 80. This area is also monitored by the transmission light detector, which generates a transmission signal T.
- The peripheral component Rp is generated by light reflected from a part 95 of the frame elements 90, part 75 of the first side 24a of the measurement chamber, and the part of the elongate textile body 16 that is outside measurement window 80 but still inside illumination area 70.

A black elongate body 16 reflects less of the light incident on it than a white elongate body 16, and for a given "color darkness" an elongate body 16 with smaller radial extent reflects less than one with larger radial extent.

A variation in body thickness located in front of measurement window 80, as shown in Fig. 6, generates a strong variation ΔT in transmission and a strong variation ΔR in reflection. A variation of body color in front of the measurement window affects the transmission only weakly, i.e. ΔT ≅ 0.

Hence, the variation ΔT is a direct measure of the body thickness, irrespective of the color of elongate body 16.

A variation of body thickness as well as a variation of body color both generate a variation ΔR in reflection. Hence, from a variation ΔR alone, a variation in body thickness cannot be distinguished from a variation in body color.

In order to reliably detect color variations, therefore, the reflection signal is combined with the transmission signal in order to generate a parameter that depends on color variations ΔC only, e.g., by calculating ΔC = k1·ΔT + k2·ΔR, with suitable constants k1, k2 selected such that pure thickness variations within measurement window are compensated, i.e., do not affect ΔC. This works well because, in the situation of Fig. 6, the thickness variation is seen by both the reflection as well as the transmission sensor.

Fig. 7 shows a situation where the elongate textile body 60 has a thickness variation 17 in front of part 95 of the frame elements 90 illuminated by the reflection light source. The thickness variation 17 prevents the reflection light sensor from receiving light from the area of the frame elements 90 covered by the thickness variation 17. This reduces the measured reflection, namely its peripheral component Rp, but it does not affect the measured transmission T, therefore generating erroneous color variation signal ΔC, e.g., when calculated as described above.

This erroneous color variation signal is a function of the difference of the average reflections of the frame elements 90 and elongate textile body 60. For highly reflective (e.g., whitish) yarns and conventional, highly reflective support bodies 14, this error is small. However, when measuring on a darker, less reflective yarn, this erroneous color variation signal becomes large.

This makes the optical detection device assume that the elongate body 16 has a darker color.

However, when using frame elements 90 as described above, in particular with reduced reflectance and/or with a width of at most 1.5 mm in the direction X and/or with an inclined surface facing the measurement chamber, reduce the amount of reflection light reflected from the frame elements 90 onto the reflection light detector and therefore reduce the effect of thickness variations 17 in the peripheral region for color determination of the elongate body 16.

The amount of reflection light reflected from the frame elements 90 may also be reduced by a surface layer 91 on the frame elements 90 as shown, for example, in Fig. 2.

### Illumination

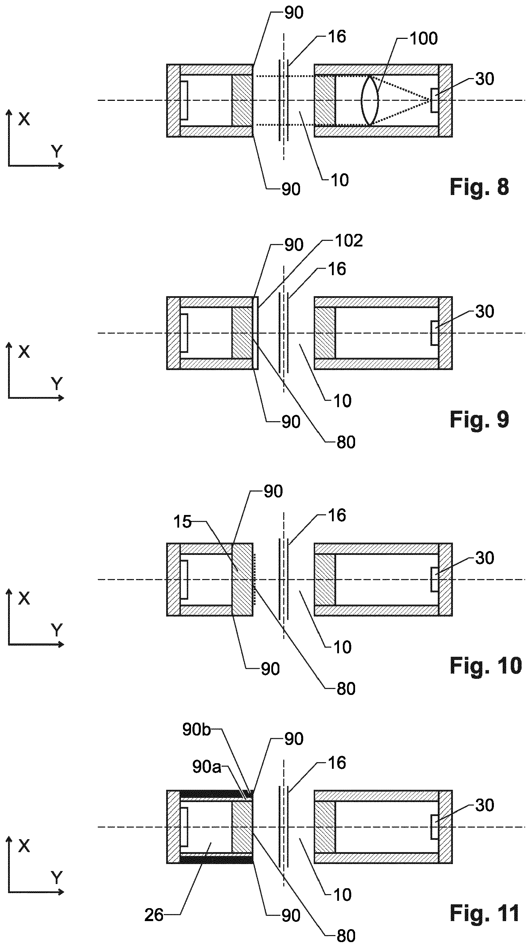

As shown in Fig. 8, in order to further reduce the influence of the frame elements 90 on the signal of the reflection sensor, the light from the reflection light source may be collimated as it enters measurement chamber 10. To this effect, for example, suitable collimation optics, such as a lens 100, may be arranged between the reflection light source 30 and measurement chamber 10. In that case, the amount of light that hits the frame elements 90 and is returned to the reflection detector (not shown in Fig. 8) is reduced.

### Reflector

In another embodiment shown in Fig. 9, a reflector 102 may be arranged at the "first" side the measurement chamber 10, with the reflector 102 covering at least the measurement window 80 and, optionally, the frame elements 90. The reflector 102 is sufficiently transparent to allow to for the measurement of the transmission sensor. Advantageously, the reflector 102 has a transmission of at least 10% at the spectral range of the transmission sensor. On the other hand, though, the reflector has a reflectance of at least 33%, in particular of at least 50% at the spectral range of the reflection sensor. This increases the amount of light reflected from measurement window 80 as compared to the amount of light reflected from the frame elements 90, thereby reducing the influence of the frame elements 90 on the signal measured by the reflection sensor.

### Multi-section frame elements

Fig. 11 shows yet another embodiment. Here, the frame elements 90 comprise, along direction X, several sections, with an inner section 90a adjacent to measurement window 80 and an outer section 90b adjacent to the inner section 90a. The inner section 90a has higher reflectance (over an emission spectrum of the reflection light source) than an outer section 90b. This allows to reduce the total reflectivity of the frame elements as seen by the reflection sensor. For a very low reflectivity, inner section 90a may have smaller extension, along direction X, than outer section 90b.

Inner section 90a lines at least one wall of light channel 26 behind measurement window 80, thereby providing it with good reflectance. Outer section 90b improves the mechanical stability of the device and/or allows to position the device against other devices along direction X.

One or both frame elements 90 may have such a multi-section design.

### Notes

As mentioned, each light source 30a - 30c may comprise several light emitters, such as several light emitters at different wavelengths, in order to perform measurements at different spectral regions. Similarly, each light detector 32a - 32c may comprise several light sensors sensitive to different spectral regions.

The light sources 30a - 30c may comprise LEDs. For example, each light source may comprise several LEDs of different colors.

The light detectors 32a - 32c may, e.g., comprise photodiodes.

In the above embodiments, the light sources 30a - 30c and light detectors 32a - 32c are arranged on the first and second peripheral sides 20a, 20b on carrier plates 34a, 34b. Alternatively, though, some of them may, e.g., also be arranged on third and/or fourth peripheral side 20c, 20d.

In the shown embodiments, for example embodiment 1, the window element 15 and measurement window 80 have, along direction X, the same spatial extent.

However, in any of the above embodiments, window element 15 may have a larger spatial extent, along direction X, than measurement window 80. This is shown in Fig. 10 where the frame elements 90 may be covered by window element 15. However, even in this case, the frame elements 90 are, when seen along direction X, still adj acent to measurement window 80. (The extent of measurement window 80 along direction X is shown with a dotted line in Fig. 10.)

As mentioned, the second sub-bodies 14b form the frame-elements 90, wherein the frame-elements 90 may be coated with surface layer 91. Said surface layer allows to reduce reflection on the frame-elements 90 while allowing the second sub-bodies 14b to be strongly reflective. In other words, the surface layer allows for low reflection on the frame elements while allowing high reflection in the light channels 26a-26f due to highly reflective second sub-bodies 14b.

While there are shown and described presently preferred embodiments of the invention, it is to be distinctly understood that the invention is not limited thereto but may be otherwise variously embodied and practiced within the scope of the following claims.

## Claims

1. An optical sensor device for detecting defects in an elongate textile body (16), comprising
a support body (14),
a measurement chamber (10) for receiving the elongate textile body (16) running along a direction X, wherein the measurement chamber (10) is,
- along the direction X, open on opposite entry (22a) and exit sides (22b) and
- along a direction Y transversal to the direction X, confined by the support body (14) on a first (24a) and second side (24c),
at least one measurement window (80) arranged on the first side (24a), wherein, along the direction X, the support body (14) comprises frame elements (90) adjacent to the measurement window (80),
an optical transmission sensor comprising
- a transmission light source arranged to send light through the measurement chamber (10) and
- a transmission light detector arranged to receive light from the transmission light source after traversing the measurement chamber (10) and the window (80), wherein the measurement chamber (10) is arranged between the transmission light source and the transmission light detector,
an optical reflection sensor comprising
- a reflection light source arranged to send light into the measurement chamber (10) and
- a reflection light detector arranged to receive light from the transmission light source after being reflected in the measurement chamber (10),
wherein the reflection light source and the reflection light detector are both arranged at the second side (24c), and
wherein the reflection light source is positioned to illuminate at least an illuminated section (95) of the frame elements (90), and the reflection light detector is positioned to receive light from the illuminated section (95).

2. The optical sensor device of claim 1, wherein the optical reflection sensor comprises at least two reflection light detectors (32b, 32c), in particular wherein at least two reflection light detectors (32b, 32c) are arranged on the second side (24c).

3. The optical sensor device of any of the preceding claims, wherein the frame elements (90) have a reflectance of at most 80 %, in particular at most 50 %, more particularly at most 20 %, over an emission spectrum of the reflection light source.

4. The optical sensor device of any of the preceding claims, wherein an average reflectance of the frame elements (90) over a spectral range ranging from 400 nm to 780 nm is at most 80 %, in particular at most 50 %, more particularly at most 20 %.

5. The optical sensor device of any of the preceding claims, wherein an average reflectance of the frame elements (90) over an emission spectrum of the reflection light source is at most 80 %, in particular at most 50 %, more particularly at most 20 %.

6. The optical sensor device of any of the preceding claims, wherein the frame elements (90) have a bulk material and a surface layer (91), wherein the surface layer (91) has an average reflectance of at most 80 %, in particular at most 50 %, more particularly at most 20 %, over an emission spectrum of the reflection light source.

7. The optical sensor device of any of the preceding claims, wherein the transmission light sensor is arranged on the first side (24a).

8. The optical sensor device of any of the preceding claims, wherein the frame elements (90) have a width, in the direction X, of at most 1.5 mm, in particular of at most 0.5 mm.

9. The optical sensor of any of the preceding claims, wherein at least one of the frame elements (90) has an inclined surface facing the measurement chamber (10), with said inclined surface being positioned to be illuminated by the reflection light source, in particular by at least one of the light sources, wherein said inclined surface is non perpendicular to the direction Y, in particular forms an angle between 2 to 88 degrees to the direction Y, more particularly forms an angle between 10 to 70 degrees to the direction Y.

10. The optical sensor of claim 9 wherein the inclined surface forms at least 20%, in particular of at least 50%, of a total surface of the frame elements (90) positioned to be illuminated by the reflection light source.

11. The optical sensor device of any of the preceding claims,
wherein
the optical reflection sensor comprises at least two, in particular at least three reflection light sources having different emission spectra, in particular having non overlapping emission spectra, or
wherein the optical reflection sensor comprises at least one reflection light source having a FWHM spectral width of at least 200 nm.

12. The optical sensor device of any of the preceding claims
wherein the support body (14) forms walls of channels (26a-26f) extending between the chamber (10) and at least some of the light sources and light detectors, wherein a reflectance of said wall over an emission spectrum of the reflection light source is higher than a reflectance of said frame elements (90) over the emission spectrum of the reflection light source.

13. The optical sensor of claim 12 wherein the reflectance of the walls is at least twice as large as the reflectance of the frame elements (90).

14. The optical sensor of any of the preceding claims wherein at least one of the frame elements is formed by an inner section (90a) adjacent to the measurement window (80) and an outer section (90b) adjacent to the inner section (90a), with the inner section (90a) having higher reflectance than the outer section (90b).

15. Use of the optical sensor device according to any of the preceding claims to perform measurements on an elongate textile body (16) having an average reflectance of at most 80% over an emission spectrum of the reflection light source, in particular an average reflectance of at most of at most 50 %, more particularly at most 20 %.

16. Use of the optical sensor device according to claim 15, wherein the elongate textile body (16) has an average reflectance of at most 80% over an emission spectrum of any one of the reflection light sources of the optical reflection sensor, in particular an average reflectance of at most of at most 50 %, more particularly at most 20 %.
